Europäisches Patentamt

European Patent Office

Office européen des brevets

(19)

(11) Publication number: **0 168 662**
A1

# EUROPEAN PATENT APPLICATION

(12)

(21) Application number: 85107572.1

(22) Date of filing: 19.06.85

(51) Int. Cl.⁴: **C 12 N 15/00**, C 12 P 19/34,
C 12 N 5/00, C 12 N 7/00,
C 12 P 21/00, C 07 K 3/00,
C 07 K 15/14
// A61K39/245 , C12R1:19

(30) Priority: 19.06.84 JP 127152/84

(43) Date of publication of application: 22.01.86
Bulletin 86/4

(84) Designated Contracting States: AT BE CH DE FR GB IT
LI NL SE

(71) Applicant: Juridical Foundation The
Chemo-Sero-Therapeutic Research Institute, 668, Okubo
Shimizu-machi, Kumamoto-shi Kumamoto-ken (JP)

(72) Inventor: Eto, Tatsuo, 29-27, Tsunoura-machi,
Kumamoto-shi Kumamoto-ken (JP)
Inventor: Nozaki, Chikateru, 622-8, Shinchi
Shimizu-machi, Kumamoto-shi Kumamoto-ken (JP)
Inventor: Kino, Yoichiro, 2-142, Musashigaoka,
Kumamoto-shi Kumamoto-ken (JP)
Inventor: Makizumi, Keiichi, 137, Yamamuro
Shimizu-machi, Kumamoto-shi Kumamoto-ken (JP)
Inventor: Ohtomo, Nobuya, 6-22-29, Shimasaki,
Kumamoto-shi Kumamoto-ken (JP)

(74) Representative: Vossius Vossius Tauchner Heunemann
Rauh, Siebertstrasse 4 P.O. Box 86 07 67,
D-8000 München 86 (DE)

(54) Recombinant DNA inserted with herpes simplex virus gene, mammalian cells transformed with said recombinant DNA, and method for the production of herpes simplex virus proteins.

(57) Novel recombinant DNA obtained by inserting a herpes simplex virus gene (e.g. HSVgB) into an E. coli plasmid inserted with TK gene, novel transformed mammalian cells obtained by transforming mammalian cells, particularly TK⁻ mammalian cells, with said recombinant DNA, and method for the production of herpes simplex virus proteins (e.g. gB) in a high yield and high purity by culturing said transformed mammalian cells. The herpes simplex virus proteins are useful for the preparation of HSV vaccine and diagnostic reagents for herpes simplex infections.

Our Ref: T 847 EP
Juridical Foundation The Chemo-Sero-
Therapeutic Research Institute, Japan
Case: 610061

0168662

VOSSIUS · VOSSIUS · TAUSCHNER
HEUNEMANN · RAUH
PATENTANWÄLTE
SIEBERTSTR. 4, 8000 MÜNCHEN 80
TEL. (089) 47 40 75

June 19, 1985

RECOMBINANT DNA INSERTED WITH HERPES SIMPLEX
VIRUS GENE, MAMMALIAN CELLS TRANSFORMED WITH
SAID RECOMBINANT DNA, AND METHOD FOR THE
PRODUCTION OF HERPES SIMPLEX VIRUS PROTEINS

The present invention relates to a novel recombinant DNA inserted with herpes simplex virus gene, mammalian cells transformed with said recombinant DNA, and a method for the production of herpes simplex virus proteins, wherein a genetic engineering technique is applied to the production of the desired herpes simplex virus proteins having a high purity which are useful for the production of herpes simplex vaccine effective for the prophylaxis of herpes simplex virus infections.

More particularly, the present invention provides a novel recombinant DNA which is obtained by inserting a gene of herpes simplex virus (hereinafter, referred to as "HSV"), specifically HSVgB gene, into an Escherichia coli plasmid derivative, i.e. a plasmid vector produced by inserting a gene of thymidine kinase (hereinafter, referred to as "TK") into an E. coli plasmid, novel transformed mammalian cells which are produced by transforming mammalian cells, specifically TK deficient mammalian cells (e.g. mouse LTK⁻ cells) with said recombinant DNA, and a method for the production of HSV proteins, particularly HSV membrane proteins in a high yield and high purity by culturing said transformed mammalian cells.

Technical Background and Prior Art

In developed countries, population having immunity against HSV has recently decreased, and hence, serious problems increase in HSV infections such as herpes genitalis, neonatal herpes infection and herpes encephalitis in these countries. In order to prevent such HSV infections, vaccine is useful, and there have already been proposed some vaccines such as a          vaccine comprising attenuated HSV and inactivated vaccine containing HSV DNA. It is known, however, that HSV includes some problems such as latent infection and carcinogenicity, and the conventional attenuated vaccine and inactivated vaccine include such side effects and hence are not preferable in practical viewpoint.

Cells infected by HSV produce several glycoproteins [e.g. gB, gC, gD, gE, etc., the nomenclature of gA and gB has been standardized as "gB" in International Herpes Virus Workshop (Oxford, England) in 1983]. Since it has been found that these glycoproteins show important function as an antigen for inhibiting HSV infection, various studies have been done on component vaccines comprising these glycoproteins. For instance, Cappel et al. have reported that glycoproteins extracted from HSV-infected cells or virus particles are effective as an antigen for preventing infection of HSV [cf. Cappel et al., Arch. Virol., 73, 61 (1982)]. However, the component vaccine comprising such glycoproteins extracted from HSV-infected cells or virus particles contains a multitude of proteins derived from the host cells and hence has a problem of side effect due to these extra proteins. In order to obtain suitable component vaccine having no side effect, it is necessary to obtain highly purified glycoproteins. The present inventors aimed at gB which is one of the glycoproteins and have experimentally confirmed that the highly purified gB is very effective in mice [cf. Kino, Cellular Technology, 3, 120 (1984)].

The glycoprotein gB is usually produced by inoculating a virus into culture cells and then culturing the cells. However, this method is troublesome in the procedure because of handling infectious material and in the complicated steps, and further, it is impossible to confirm the complete removal of viral DNA carrying carcinogenic sequences.

Thus, it is very difficult to produce a practically safe component vaccine from natural glycoprotein gB.

Brief Summary of the Invention

The present inventors have intensively studied on safe vaccine containing no carcinogenic gene by isolating the gB gene and expressing said gene in mammalian cells and have now accomplished in the isolation of gB gene and the expression of said gene.

An object of the present invention is to provide a

novel recombinant DNA inserted with HSV gene which is suitable for transforming mammalian cells. Another object of the invention is to provide mammalian cells transformed by said recombinant DNA (e.g. transformed mouse L cells). A further object of the invention is to provide a method for the production of HSV proteins by using said transformed mammalian cells. These objects and advantages of the present invention will be apparent to persons skilled in the art from the following description.

## Brief Description of the drawing

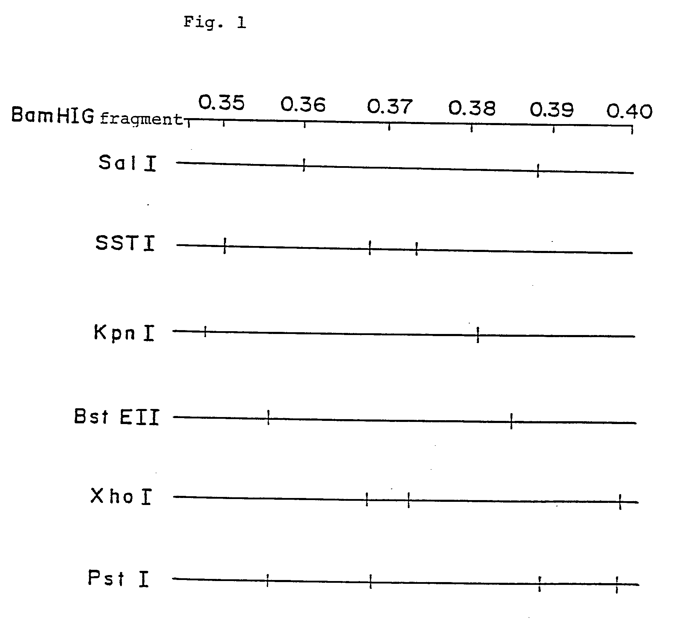

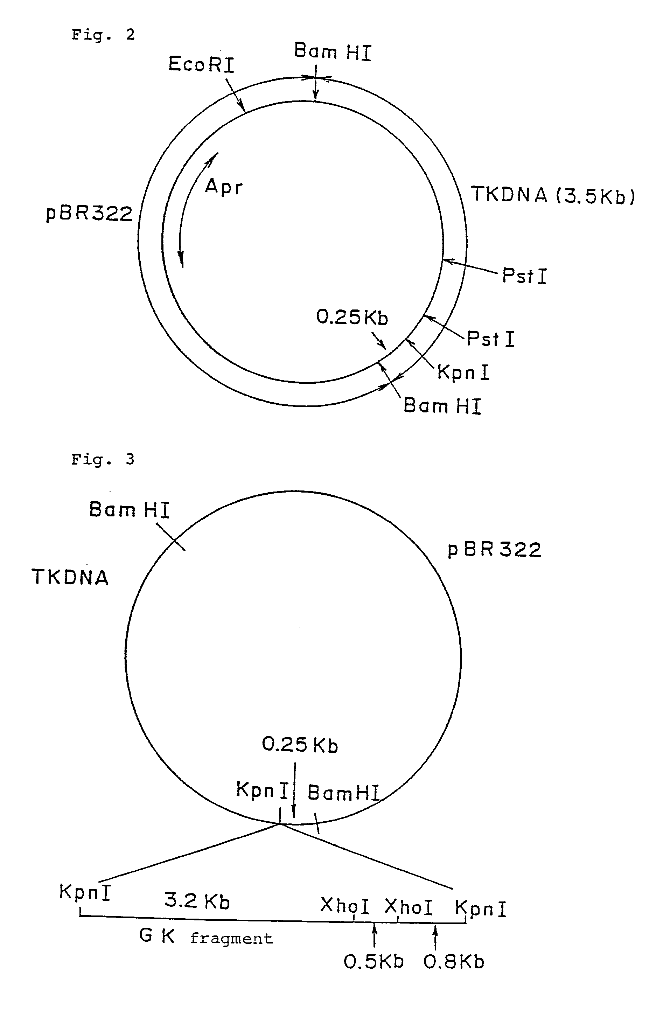

Fig. 1 shows a map of BamHI G fragment of HSV DNA and cleavage sites thereof with various restriction enzymes. Fig. 2 shows a structure of a recombinant DNA of E. coli plasmid pBR 322 and a TK gene from HSV DNA. Fig. 3 shows one embodiment of the recombinant DNA of the present invention which is produced by inserting GK fragment containing HSVgB gene into a vector consisting of E. coli plasmid pBR 322 containing a TK gene.

## Detailed Description of the Invention

The desired recombinant DNA of the present invention can be produced by using as a vector derivatives of E. coli plasmid, e.g. a DNA obtained by inserting TK gene into E. coli plasmid and then recombining an HSV gene (i.e. HSVgB gene) into said vector. The recombinant DNA thus produced is used for transformation of mammalian cells, preferably TK deficient mammalian cells (e.g. mouse LTK⁻

cells).  When the transformed mammalian cells are cultured
under suitable culture conditions / they give the desired HSV
proteins (i.e. HSVgB).

The recombinant DNA, transformed mammalian cells
and the production of HSV proteins of the present invention
are illustrated in more detail below.

(1)  HSVgB gene

The HSVgB gene used in the present invention is
HSVgB DNA which is cloned in E. coli.

The HSV DNA is a DNA having a molecular weight of
about 160 K dalton and HSVgB gene is present in a fragment
having about 8 kb (0.345 - 0.399 map units) which is
obtained by treating HSV DNA with a restriction enzyme:
BamHI, said fragment being hereinafter referred to as "BamHI
G fragment".  This BamHI G fragment has several cleavage
sites with various restriction enzymes (e.g. Sal I, Sst I,
Kpn I, Bst EII, Xho I, Pst I) as is shown in the accompanying Fig. 1.  Thus, the HSVgB gene used in the present
invention includes BamHI G fragment and further various
fragments obtained by cleaving the BamHI G fragment with
various restriction enzymes, preferably a fragment having
about 4.5 Kb (0.348 - 0.381 map units) which is obtained by
treating BamHI G fragment with Kpn I (hereinafter, referred
to as "GK fragment").

These fragments can be prepared by cleaving HSV
DNA with BamHI, separating  thus cleaved BamHI G fragment  by

agarose gel electrophoresis, amplifying said fragment by cloning in E. coli, preferably followed by treating with a restriction enzyme, e.g. Kpn I.   The fragments thus obtained are used for the subsequent construction of the recombinant DNA as they stand.

(2)   Derivatives of E. coli plasmid

The E. coli plasmid derivatives are used as a vector and are recombinant DNAs which are produced by inserting a TK gene from HSV DNA into an E. coli plasmid, for instance, a recombinant DNA obtained from E. coli plasmid pBR322 and HSV DNA which has a structure as shown in the accompanying Fig. 2 [cf. Florence Colbere-Garapin, 3rd General Meeting of ESACT, Oxford 1979, Develop. Biol. Standard, 46, 75-82 (1980)].

That is, TK gene (3.5 Kb) is inserted into the BamHI site of E. coli plasmid pBR322 to give the desired recombinant DNA which has an ampicillin resistant gene (Apr).   The antibiotic resistant gene is effective as a selection marker for the transformed cells disclosed hereinafter, and includes tetracycline resistant gene, kanamycin resistant gene, chloramphenicol resistant gene, etc.

These vectors are combined with an HSV gene to give the desired recombinant DNA, wherein the vectors are used in the form of a fragment which is obtained by cleaving the vector with a suitable restriction enzyme, e.g. Kpn I.

(3)  Construction of recombinant DNA (HSV gene-
expression plasmid)

The above vector i.e. the E. coli plasmid
derivative containing TK gene is treated with a suitable
restriction enzyme which does not inactivate the TK gene
(e.g. Kpn I) to give a fragment.  The fragment thus obtained
is reacted with a fragment containing the above HSVgB gene
in an appropriate ratio to give the desired recombinant
DNA.  Depending on the ratio of the fragment to the vector,
there are constructed various recombinant DNAs wherein 1 or
several fragments of HSVgB DNA are combined with/ 1 fragment of
the vector.  Preferred recombinant DNA is inserted with 1
or 2 fragments of the HSVgB DNA per 1 fragment of the
vector.

The recombinant DNA thus obtained has a structure
as shown in the accompanying Fig. 3.  The recombinant DNA
shown in Fig. 3 has one Kpn I fragment of HSVgB DNA (i.e. GK
fragment) at the Kpn I site of the vector DNA.

(4)  Transformation of mammalian cells

Mammalian cells are transformed with said
recombinant DNA which carries the genetic information for
HSVgB.

The mammalian cells include preferably TK$^-$ mammalian cells, particularly mouse LTK$^-$ cells.

The procedure of transformation is explained in
more detail in case of using mouse LTK$^-$ cells.

Mouse LTK⁻ cells are cultured in a Dulbecco's modified Eagle medium (hereinafter, referred to as "DMEM") supplemented with 10 % calf serum [cf. Dulbecco, B. & Freeman, G.; Virology, 8, 396, 1956] and thereto is added a solution of the recombinant DNA in an aqueous calcium phosphate solution, and the mixture is allowed to stand at room temperature for a few or several tens of minutes, usually about 30 minutes.  To the resulting mixture is added additional DMEM, and the mixture is cultured for 4 to 5 hours.  After substituting by  new DMEM, the mixture is further cultured for 12 to 24 hours.  The resulting culture is further cultured in a medium containing hypoxanthine (15 μ g/ml), aminopterin (1 μg/ml) and thymidine (5 μg/ml) (hereinafter, referred to as "HAT medium") [cf. Littlefield; J. Proc. Natl. Acad. Sci. USA, 72, 3961-3965, 1963] to give the desired transformed mouse L cells.  In the above transformation procedure, the starting mouse LTK⁻ cells are deficient in TK gene and hence can not grow in HAT medium, but on the other hand, the transformed mouse L cells inserted with TK gene are capable of synthesis of thymidine kinase and hence can grow in HAT medium.  Accordingly, only the desired transformed  cells can selectively be isolated by culturing in the above HAT medium.

(5)  Culture of transformed mammalian cells and production of HSVgB

The transformed mammalian cells obtained above are cultured in an appropriate medium in a usual manner to produce a large amount of HSVgB within the grown cells. The isolation of the HSVgB thus produced can be done by separating the grown mammalian cells, treating the cells with a surfactant (e.g. Triton X-100, Nonidet P-40, etc.) or an organic solvent (e.g. carbon tetrachloride, diethyl ether, etc.) to fracture the cell membrane, followed by purifying the gB by a conventional method for purifying proteins. For instance, the gB-containing extract is passed through a column packed with a gel bound with anti-gB antibody and then eluting the gB with 3M KSCN.

The gB thus obtained has the same immunological properties as those of the natural gB obtained from HSV-infected cells and can be used for the preparation of HSV vaccine and diagnostic reagents.

The recombinant DNA, transformed mammalian cells, and the production of HSVgB of the present invention are illustrated by the following example, but should not be construed to be limited thereto.

Example

(1) Preparation of HSV-1 DNA:

Vero cells (about $5 \times 10^8$ cells) are infected with 0.5-1 PFU/cell of HSV type 1 (HSV-1) KOS strain and the cells are cultured at 37°C for 20-24 hours. When the cells are sufficiently modified, the culture mixture is centrifuged at 30,000 r.p.m. for 1 hour to separate the infected cells and the supernatant, and thereby pellets of infected cells (2-3 ml) are isolated.  The pellets are suspended in a phosphate buffered saline solution (hereinafter, referred to as "PBS") (pH 7.2, 6 ml).  The suspension is subjected to ultrasonic treatment (9 KHz, 200 W, for 5 minutes) or to freezing - thawing [repeating three times freezing at -50°C (with acetone-dry ice) and thawing at 37°C] to fracture the cells, and the cell residue is removed by centrifugation at low speed (3,000 r.p.m., 20 minutes).  The solution thus obtained is layered on a glycerol cushion (5 %, 40 %) and then centrifuged at 35,000 r.p.m. for one hour.  The pellets (0.5-1 ml) thus obtained are suspended in PBS (1-2 ml) and are treated with DNase (10 µg/ml) and RNase (0.3 mg/ml) at 37°C for one hour, and to the reaction mixture is added 1/5 volume of 5 x STEP [a mixture of 0.5 % SDS (sodium dodecyl-sulfate), 50 mM Tris-HCl (pH 7.5), 0.4 M EDTA, and 0.1 % proteinase K], and the mixture is reacted at 50°C for 30 minutes.  The resulting solution is extracted with an equi-volume of phenol, phenol-chloroform (1 : 1) and chloroform in this order to give an aqueous layer containing DNA.

The aqueous layer is dialyzed against TE buffer (20 mM Tris-HCl, 1 mM EDTA, pH 7.5), and thereto is added cold ethanol to precipitate the DNA.  The DNA is separated by filtration, dried in vacuum, and then dissolved in aqueous cesium chloride ($R_f$ 1.3885, incorporated with ethidium bromide (0.04 %) and lauroyl sarcosinate (0.4 %), 5 ml). The mixture is centrifuged at 4,000 r.p.m. for 72 hours to form a band of HSV-1 DNA. The band is recovered and is washed with isopropyl alcohol to remove ethidium bromide, and then dialyzed against TE buffer. Cold ethanol is added thereto to precipitate HSV-1 DNA.

(2) Cloning of BamHI G fragment of HSV-1 DNA:

The HSV-1 DNA obtained above (about 100 µg) is treated with a restriction enzyme BamHI in a mixture (0.75 ml) of 73 mM Tris-HCl (pH 8.0), 7 mM $MgCl_2$, 100 mM NaCl and 2 mM 2-mercaptoethanol at 37°C for 6 hours, and then each fragment is separated by 0.7 % agarose electrophoresis to cut out a gel corresponding to G fragment (0.345-0.399 map units), from which the G fragment is electrophoretically recovered.

pBR322 plasmid (1/10 mole) obtained by cleavage with a restriction enzyme BamHI is reacted with the above G fragment (about 2 µg) in a mixture of 50 mM Tris-HCl (pH 7.9), 10 mM $MgCl_2$, 20 mM dithiothreitol, and 1 mM ATP by using T4 DNA ligase at 16°C for about 16 hours.

The above reaction mixture is added to a liquid of E. coli (0.1 ml) which is prepared by treating a culture broth of E. coli χ1776 (cf. Curtiss, R. III, "Molecular Cloning of Recombinant DNA" ed. Scott, W. A. and Werner, R., page 99, Academic Press, 1977) by the procedure as described in Norgard, M. V., Gene, 3, 279 (1978), and the mixture is mixed well and allowed to stand at 0°C for 45 minutes. The mixture is applied onto an agar plate containing ampicillin (100 μg/ml) and then incubated at 37°C overnight. The resulting colonies are applied onto both an agar plate containing ampicillin (100 μg/ml) and an agar plate containing tetracycline (100 μg/ml), and the colonies which grow only on the agar plate containing ampicillin are selected. pBR322 has an ampicillin-resistant gene and a tetracycline-resistant gene, but when it is inserted with HSV-1 DNA fragment at the BamHI site of the tetracycline-resistant gene, it loses the tetracycline-resistance. Accordingly, the selected colonies contain the recombinant DNA pBR322-HSV which carries the BamHI G fragment as an insertion.

From the colonies thus selected, a plasmid is prepared by the procedure as described by K. Matsubara (J. Virol., 16, 479, 1975). The plasmid thus prepared is subjected to cleavage pattern analysis by treating with various restriction enzymes (e.g. BamHI, Bst EII, Kpn I, Sal I, Sst I, Xho I) to obtain a recombinant DNA of pBR322-BamHI G fragment wherein BamHI G fragment of HSV-1 DNA is inserted into pBR322 (hereinafter, referred to as "pBR322-G").

(3) Preparation of recombinant DNA (pBR322- TK gene - GK fragment):

The pBR322-G obtained above is cleaved by treating it with / restriction enzyme Kpn I in a mixture of 6 mM Tris-HCl (pH 7.5), 6 mM NaCl, 6 mM MgCl$_2$ and 6 mM 2-mercaptoethanol, and from the resulting DNA fragments, there is obtained GK fragment (0.348-0.381 map units) in the same manner as in the preparation of G fragment as mentioned above.

The above GK fragment is bound at the Kpn I cleavage site with a previously cloned pBR322 - TK DNA (i.e. a recombinant DNA prepared by binding BamHI cleavage site of pBR322 with Q fragment obtained by cleaving HSV DNA containing TK gene with BamHI) [cf. Construction and Characterization of A Recombinant Plasmid Encoding The Gene for the Thymidine Kinase of Herpes Simplex type 1 virus, Enquist et al.,     Gene, 7, 335-342, 1979] to give a recombinant DNA of pBR322 - TK gene - GK fragment.  The preparation of the recombinant DNA is carried out by subjecting the ampicillin-resistant colonies prepared in the same manner as in the preparation of the above pBR322-G to colony hybridization using $^{32}$P-labeled GK fragment as a probe and then selecting the desired colony [cf. "Protein·Nucleic acid· Enzyme", 26, No. 4, Genetic Engineering Procedure, 575-579 (1983)].  The plasmid thus prepared is cleaved by various restriction enzymes (e.g. BamHI, Xho I, Sst I, Kpn I) in order to determine the restriction map, by which the insertion and direction of GK fragment are characterized.

(4)  Transformation of mouse LTK⁻ cells:

The recombinant DNA of pBR322 - TK gene - GK fragment obtained above is cleaved by a restriction enzyme BamHI.  The fragment thus obtained (pBR322 - TK - GK fragment) (2 µg) and LTK⁻ DNA (carrier DNA) (2 µg) are
dissolved in 130 mM $CaCl_2$ (200 µl) and thereto is added 2 x
Hepes solution (which consists of NaCl 16 g/l, KCl 0.74 g/l,
$NaHPO_4 \cdot 2H_2O$ 0.25 g/l, dextrose 2 g/l, N-(2-hydroxyethyl)-
piperazine-N-(2-ethanesulfonic acid) 10 g/l, pH 7.11) (200
µl), and the mixture is allowed to stand at room temperature
for 30 minutes. The resulting precipitates are well
suspended, and then, the mixture is added dropwise to an
about 60 % single layer of mouse LTK⁻ cells (about $10^5$
cells/flask) in a flask. The flask is kept at 37°C for one
hour in order to make the mixture absorbed into the cells,
and thereto is added DMEM (5 ml), and the mixture is
incubated under 5 % $CO_2$ at 37°C for 4 hours. After removing
DMEM by suction, a Hepes solution containing 15 %
dimethylsulfoxide is added to the mixture, and the mixture
is stirred at room temperature for 4 minutes. The mixture
is washed with DMEM twice, and then new DMEM (5 ml) is
added, and the mixture is incubated under 5 % $CO_2$ at 37°C
for 24 hours. The medium is exchanged to HAT medium, and
the incubation is continued while the medium is exchanged
with a new HAT medium every two to three days. After about
two weeks, the colonies of HAT-resistant cells (LTK⁺ cells)
are collected to give the desired transformed cells.

(5) Production of HSV-1 HSVgB

The transformed mouse L cells (LTK⁺ cells)
obtained above are defatted by treating with carbon tetrachloride for 10 minutes and are reacted with a monoclonal antibody against HSV-1 surface glycoprotein gB (as a primary antibody). After washing and drying, the cells are further reacted with a fluorescent pigment-labelled anti-mouse IgG (as a secondary antibody), and then washed, dried and encapsulated.

According to observation by a fluorescence microscope, a specific antibody against gB was observed within cytoplasm of the transformed cells.

Besides, a solution of the transformed cells in 1 % Triton X-100 was added to a plate having 96 wells wherein a monoclonal antibody against gB was immobilized, and they were reacted at 37°C for one hour. After washing, a monoclonal antibody against gB which was bound with peroxidase was added, and the mixture was reacted at 37°C for one hour. After washing, ortho-phenylenediamine (a substrate for peroxidase) was added to the reaction mixture, by which coloring was observed and thereby the presence of gB was confirmed.

Moreover, a solution of the transformed cells in 1 % Triton X-100 was subjected to SDS-electrophoresis and then blotted on nitrocellulose. Thereafter, the cells were reacted with a monoclonal antibody against gB. After washing, the cells were further reacted with an anti-mouse IgG antibody bound with peroxidase. After washing, diaminobenzidine (a substrate) was added to the reaction mixture, by which there was observed a gB-like pattern in the solution of HSV-1-infected LTK⁻ cells in 1 % Triton X-100.

The transformed cells containing produced gB were intraperitoneally administered to mice (BALB/c). After several weeks, blood serum of the mice was observed. As a result, there was observed a significant increase of neutralizing antibodies against HSV-1.

Claims

1.   A recombinant DNA which comprises a herpes simplex virus gene recombined with a vector consisting of an Escherichia coli plasmid inserted with thymidine kinase gene.

2.   The recombinant DNA according to claim 1, wherein the vector is a recombinant DNA which is prepared from E. coli plasmid pBR322 and a thymidine kinase gene from a herpes simplex virus DNA.

3.   The recombinant DNA according to claim 1, wherein the herpes simplex virus gene is a herpes simplex virus gB gene.

4.   The recombinant DNA according to claim 3, wherein the gB gene is a BamHI G  fragment having about 8 kb (0.345-0.399 map units) which is prepared by treating herpes simplex virus DNA with the restriction enzyme BamHI.

5.   The recombinant DNA according to claim 3, wherein the gB gene is GK fragment (0.348-0.381 map units) which is prepared by treating herpes simplex virus DNA with the restriction enzyme BamHI and further treating the resulting BamHI G fragment with  the restriction enzyme  Kpn I.

6.   The recombinant DNA according to any one of claims 1 to 5, wherein one or several herpes simplex virus genes are recombined.

7.   The recombinant DNA according to claim 6, wherein the number of the herpes simplex virus genes to be recombined is one or two.

8. A transformed mammalian cell which is prepared by transforming a mammalian cell with the recombinant DNA as set forth in claim 1.

9. The transformed mammalian cell according to claim 8, wherein the mammalian cell is a thymidine kinase deficient mammalian cell.

10. The transformed mammalian cell according to claim 8, wherein the mammalian cell is a mouse LTK$^-$ cell.

11. A method for the production of herpes simplex virus proteins, which comprises culturing the transformed mammalian cell as set forth in claim 8 to produce herpes simplex virus glycoproteins and collecting the proteins.

12. The method according to claim 11, wherein the herpes simplex virus proteins are herpes simplex virus membrane proteins gB.

13. A method for the production of a recombinant DNA inserted with a herpes simplex virus gene, which comprises treating an E. coli plasmid containing TK gene with a restriction enzyme which does not inactivate the TK gene, and reacting the fragment thus prepared with a fragment containing a herpes simplex virus gene.

14. The method according to claim 13, wherein the fragment containing a herpes simplex virus gene is a BamHI G fragment having about 8 kb (0.345-0.399 map units) which is prepared by treating herpes simplex virus DNA with the restriction enzyme BamHI.

15.   The method according to claim 13, wherein the fragment containing a herpes simplex virus gene is GK fragment (0.348-0.381 map units) which is prepared by treating herpes simplex virus DNA with the restriction enzyme BamHI and further treating the resulting BamHI G fragment with the restriction enzyme Kpn I.

16.   The method according to claim 13, wherein one fragment of the E. coli plasmid containing TK gene is reacted with one or several fragments containing a herpes simplex virus gene.

17.   The method according to claim 16, wherein the fragment containing a herpes simplex virus gene is used one or two per one fragment of the E. coli plasmid containing TK gene.

18.   A method for preparing transformed mammalian cells transformed by a recombinant DNA inserted with herpes simplex virus gene, which comprises applying a recombinant DNA inserted with herpes simplex virus gene to mammalian. cells in a medium and culturing the mixture.

19.   The method according to claim 18, wherein the mammalian cells are $TK^-$ mammalian cells.

20.   The method according to claim 18, wherein the mammalian cells are mouse $LTK^-$ cells.

0168662

Fig. 1

Fig. 2

Fig. 3

## DOCUMENTS CONSIDERED TO BE RELEVANT

EP 85107572.1

| Category | Citation of document with indication, where appropriate, of relevant passages | Relevant to claim | CLASSIFICATION OF THE APPLICATION (Int Cl 4) |
|---|---|---|---|
| A | EP - A2 - 0 100 521 (BEHRINGWERKE AKTIENGESELLSCHAFT)<br>  * Claims 1,4,8 *<br>  -- | 1,2,8, 13,18 | C 12 N 15/00<br>C 12 P 19/34<br>C 12 N  5/00<br>C 12 N  7/00 |
| A | EP - A2 - 0 101 655 (MOLECULAR GENETICS, INC.)<br>  * Claims 1,6,10,15,22 *<br>  -- | 1,11, 13 | C 12 P 21/00<br>C 07 K  3/00<br>C 07 K 15/14 |
| A | EP - A2 - 0 074 808 (UNIVERSITY PATENTS, INC.)<br>  * Claim 1 *<br>  ---- | 1,13 | //A 61 K 39/245<br>C 12 R  1:19 |

### TECHNICAL FIELDS SEARCHED (Int Cl 4)

C 12 N

C 12 P

C 07 K

A 61 K

The present search report has been drawn up for all claims

| Place of search | Date of completion of the search | Examiner |
|---|---|---|
| VIENNA | 23-09-1985 | WOLF |